# EUROPÄISCHE PATENTANMELDUNG

(11) **EP 2 160 996 A1**
(43) Veröffentlichungstag der Anmeldung: **10.03.2010**
(21) Anmeldenummer: 08015678.9
(22) Anmeldetag: 05.09.2008
(51) Int. Cl.: A61B 19/08, A41D 13/11, A61B 19/00, A45D 44/00

(54) **Gesichtsmaske für kosmetische oder ärztliche Behandlungen**

(71) Anmelder: Baum, Jürgen, 07160 Calvia/Paguera (ES)
(72) Erfinder: Baum, Jürgen, 07160 Calvia/Paguera (ES)
(74) Vertreter: Kohlmann, Kai

(57) **Zusammenfassung**

Die Erfindung betrifft eine Gesichtsmaske (1) umfassend eine zentrale Augen-, Nasen-, Mund- und Wangenpartie sowie eine Stirn- und Kinnpartie zur abdeckung der Gesichtsfläche eines Maskenträgers mit Ausnehmungen zumindest für Mund (5) und Nase (6) sowie einer Halterung zur Befestigung der Gesichtsmaske am Kopf des Maskenträgers.
Um einen Spritzschutz für kosmetische bzw. zahnmedizinische Behandlungen zu schaffen, der preiswert herstellbar ist und sich ohne zusätzliche Befestigungsmittel am Kopf des Maskenträger sicher befestigen lässt, wird erfindungsgemäß vorgeschlagen, dass bei einer derartigen Gesichtsmaske seitlich neben der Augen-, Nasen-, Mund- und Wangenpartie Durchgänge zur Aufnahme der Ohren (9a,9b) des Maskenträgers angeordnet sind.

## Beschreibung

Die Erfindung betrifft eine Gesichtsmaske umfassend eine zentrale Augen-, Nasen-, Mund - und Wangenpartie sowie eine Stirn- und Kinnpartie zur Abdeckung der Gesichtsfläche eines Maskenträgers mit Ausnehmungen zumindest für Augen und Mund sowie einer Halterung zur Befestigung der Gesichtsmaske am Kopf des Maskenträgers.

Bei zahnärztlichen Behandlungen, wie beispielsweise bei der Zahnreinigung, kommt es zu einer Benetzung der Gesichtsfläche durch Flüssigkeiten, wie insbesondere Wasser, Reinigungspartikel sowie ggf. Blut, die während der Reinigungsbehandlung im Mundraum nach außen spritzen. Bei der Zahnreinigung wird üblicherweise auf eine Abdeckung der Gesichtsfläche verzichtet. Allenfalls werden Teilflächen des Gesichts durch Tücher abgedeckt. Auch bei herkömmlichen zahnärztlichen Behandlungen oder Operationen im Mundraum spritzen aus dem Mundraum feine Wasser-, Blut- oder Sekretpartikel, die sich auf der Gesichtsfläche und ggf. in den Haaren sowie auf der Kleidung, verteilen. In der zahnärztlichen Praxis wird jedoch lediglich die Bekleidung am Oberkörper durch ein Papiertuch nach Art einer Serviette geschützt.

Aus der DE 100 02 350 A1 ist bereits eine Gesichtsmaske zur Abdeckung der Gesichtsfläche bekannt, die jedoch nicht dem Spritzschutz bei kosmetischer oder zahnmedizinischer Behandlung dient, sondern das Aufbringen von Salben oder Cremes im Gesicht durch Temperaturerhöhung unterstützen soll. Damit diese Gesichtsmaske ein Wärme- oder Kältemittel aufnehmen kann, besteht sie aus einer ersten unteren und einer zweiten oberen Folie, die übereinander angeordnet und entlang ihres äußeren Umfangs unter Ausbildung eines Hohlraums miteinander verschweißt sind. Um den Tragekomfort, insbesondere bei längerem Aufliegen der Gesichtsmaske, zu verbessern, weist diese Ausnehmungen für Mund, Nase und Augen auf. Außerdem besitzt die bekannte Gesichtsmaske einen oder zwei Gurte zur Befestigung am Kopf des Maskenträgers, um zu verhindern, dass diese verrutscht, wenn sich der Maskenträger hinlegt oder setzt. Schließlich sind zur Stabilisierung der Form der Gesichtsmaske zwischen den beiden, den Hohlraum zur Aufnahme des Kälte- bzw. Wärmemittels bildenden Folien Schweißnähte angeordnet.

Die DE 199 30 838 A1 offenbart eine Gesichtsmaske für medizinische oder kosmetische Zwecke, deren Grundform einem menschlichen Gesicht angepasst ist. Die Maske ist zu einer Schönheitsbehandlung oder einer Pflegebehandlung oder zum Schutz der Haut während einer Behandlung außerhalb der Maskenfläche auf das Gesicht auflegbar. Diese Gesichtsmaske umfasst eine zentrale Augen-, Nasen-, Mund- und Wangenpartie sowie eine Stirn- und Kinnpartie zur Abdeckung der Gesichtsfläche. Darüber hinaus weist sie Ausnehmungen für Augen, Mund und Nase auf. Die Maske wird an dem Gesicht des Trägers mit seitlich angeordneten Haltemitteln in Form von Bügeln oder Schlaufen zur Verbindung mit den Ohren befestigt. Die Maske besteht aus einer Kombination zweier aufeinander haftender Schichten, wobei die innere hautseitige Schicht aus einem textilen Vlies und die äußere Schicht aus einem geprägten, gepressten oder gespritzten Werkstoff besteht.

Die formstabile Maske ist auf Grund des mehrlagigen Aufbaus sowie der Press- bzw. Prägeprozesse nicht preiswert herstellbar und daher für den Einweggebrauch weniger geeignet. Die Vielzahl der zur Verwendung gelangenden Materialien steht der Verwendung als Einwegartikel ebenfalls entgegen, da derartige Mischfraktionen bei der Entsorgung Probleme aufwerfen. Die verwendeten Materialien für die äußere Schicht sind auf Grund ihrer nicht vorhandenen Saugfähigkeit zudem für kosmetische bzw. zahnmedizinische Behandlungen weniger geeignet.

Ausgehend von diesem Stand der Technik liegt der Erfindung die Aufgabe zu Grunde, einen Spritzschutz für kosmetische bzw. zahnmedizinische Behandlungen zu schaffen, der als preiswerter Einwegartikel herstellbar ist, gleichwohl bei längeren Behandlungen zeitweilig die visuelle Wahrnehmung und zeitweilig deren Unterbindung ermöglicht.

Die Lösung dieser Aufgabe basiert auf dem Gedanken, einen preiswerten Einwegartikel zu schaffen, der nach Gebrauch ohne weiteres entsorgt werden kann.

Diese Aufgabe wird bei einer Gesichtsmaske der eingangs erwähnten Art im Einzelnen dadurch gelöst,
- dass die Gesichtsmaske seitlich neben der Augen-, Nasen-, Mund - und Wangenpartie Durchgänge zur Aufnahme der Ohren des Maskenträgers aufweist,
- dass die Gesichtsmaske aus einem flexiblen Material besteht
- dass an den Ausnehmungen für die Augen jeweils ein Verschluss angeordnet ist und
- dass die Ausnehmung für das Auge und der Verschluss durch eine das Auge umgebende, auf einer Teillänge unterbrochene Stanzung im Bereich der Augenpartie der Gesichtsmaske gebildet werden.

Um bei längeren Behandlungen die visuelle Wahrnehmung zu ermöglichen, weist die Gesichtsmaske die Ausnehmungen für die Augen auf. Der an den Ausnehmungen für die Augen jeweils angeordnete Verschluss, erlaubt es die Öffnungen für die Augen bei Behandlungen mit starker Spritzerbildung zeitweilig zu verschließen. Des Weiteren erlauben die Verschlüsse an den Ausnehmungen die zeitweilige Unterbindung der Sicht bei besonders sensiblen Patienten während bestimmter Phasen der Zahnbehandlung.

Die preiswerte Herstellbarkeit wird erreicht, in dem die Verschlüsse der Ausnehmungen für die Augen einstückig und aus demselben flexiblen Material wie den Rest der Gesichtsmaske hergestellt werden. Hierzu werden die Ausnehmungen für das Auge und der Verschluss durch eine in der Augenpartie angeordnete, das Auge umgebende, auf einer Teillänge unterbrochene Stanzung in der Gesichtsmaske ausgebildet. Durch die Teilstanzung verbleibt ein Steg zwischen der Augenpartie der Gesichtsmaske und dem aus der Augenpartie als Verschluss ausgestanztem Bestandteil, wobei der Steg als Scharnier dient.

Die Gesichtsmaske besteht aus einem flexiblen Material, insbesondere aus einem Zellstoff. Dabei wird unter dem Begriff Zellstoff ein glattes-, gerautes- oder genopptes flächiges Zellstoffgewebematerial verstanden, das zur Herstellung der Gesichtsmaske verwendet wird. Die Gesichtsmaske kann aus Zellstoff oder Zellstoffvlies, in einer oder mehreren Lagen bestehen. Alternativ kommen jedoch auch andere flexible Materialien, wie beispielsweise Folienmaterialien oder Materialien aus Baumwollfasern, in Betracht. Die Dicke der Zellstofflage bzw. Zellstofflagen ist flexibel und richtet sich nach der für den jeweiligen Anwendungsfall erforderlichen Flüssigkeitsaufnahme der Gesichtsmaske. Für einfache zahnärztliche Behandlungen genügen ein- bis zweilagige Gesichtsmasken mit einer Dicke des Zellstofftuches von 0,3 - 0,4 mm. Bei zeitintensiveren Behandlungen mit höherer Feuchtigkeitsbenetzung kommen insbesondere zwei- oder dreilagige Gesichtsmasken mit einer Gesamtdicke bis zu 1,5 mm in Betracht.

Vorzugsweise werden die erfindungsgemäßen Gesichtsmasken steril und einzeln verpackt. In diesem Fall lassen sie sich auch für chirurgische Behandlungen nutzen.

Die Gesichtsmaske weist seitlich neben der Augen-, Nasen-, Mund- und Wangenpartie Durchgänge zur Aufnahme der Ohren des Maskenträgers auf. Dabei wird davon ausgegangen, dass die Gesichtsfläche als vorderer Teil des Kopfes des Maskenträgers sich vom unteren Rand des Unterkiefers bis zum Haaransatz erstreckt. Man unterscheidet am Gesicht eine Stirn- und Kinnpartie sowie eine zentrale Augen-, Nasen-, Mund- und Wangenpartie. Diesen Partien sind entsprechende Bereiche der Gesichtsmaske zugeordnet. Seitlich neben der zentralen Gesichtspartie der Gesichtsmaske sind die Durchgänge zur Aufnahme der Ohren angeordnet, die die Ohren umschließen und die Gesichtsmaske sicher fixieren. Infolge dessen kann die Gesichtsmaske bei der Behandlung nicht unbeabsichtigt vom Gesicht rutschen.

Vorzugsweise befinden sich die Durchgänge zur Aufnahme der Ohren in seitlich an die Gesichtsfläche der Gesichtsmaske ansetzenden flächigen Fortsätzen. Es ist jedoch auch ohne weiteres möglich, die Durchgänge in einem über die Gesichtsfläche hinausgehenden, insbesondere um die Gesichtfläche umlaufenden Randbereich anzuordnen.

Um eine lagerichtige Befestigung der Gesichtsmaske zu gewährleisten, erstrecken sich die Durchgänge zur Aufnahme der Ohren längs der Symmetrieachse des Gesichts von der Augenpartie bis zur Mundpartie.

Die Durchgänge sind vorzugsweise ergonomisch an die Ohren angepasst, insbesondere in dem die Durchgänge zur Aufnahme der Ohren teilkreisförmig ausgestaltet sind, wobei die Kreisbögen der Ausnehmungen zu den äußeren Rändern der Gesichtsmaske weisen.

Die Befestigung der Gesichtsmaske bietet den entscheidenden Vorteil, dass die Halterung und Maske aus demselben Material, vorzugsweise aus einem Zellstoffmaterial bestehen. Infolge dessen lässt sich die erfindungsgemäße Gesichtsmaske als umweltfreundlicher Einwegartikel entsorgen, der insbesondere keinen Materialmix aufweist.

Um auch den Haaransatz bzw. die Haare wirksam gegen Spritzer zu schützen, weist die Gesichtsmaske in einer Ausgestaltung der Erfindung angrenzend an die Stirnpartie einen flächigen Fortsatz zur Abdeckung dieser Bereiche auf. Um über die Kinnpartie herunterlaufende Flüssigkeiten sowie das Absetzen von Spritzern im Halsbereich zu vermeiden, kann die Gesichtsmaske angrenzend an die Kinnpartie einen weiteren flächigen Fortsatz zur Abdeckung der Halspartie aufweisen. Die beschriebenen Fortsätze sind vorzugsweise einstückig mit dem Rest der Gesichtsmaske ausgebildet und bestehen aus demselben Material.

In einer bevorzugten Ausführungsform der Erfindung ist mindestens eine Ausnehmung auch für die Nase vorgesehen, die von einer trapezförmigen Öffnung gebildet wird, die in Richtung der Symmetrieachse der Gesichtsfläche einen Ein-/oder Anschnitt in die Gesichtsmaske bis auf Höhe der Augenpartie aufweisen kann, um eine Anpassung an unterschiedliche Nasenformen zu ermöglichen. Die Verwendung eines Anschnitts anstelle eines Einschnitts bietet den Vorteil, dass die Öffnung nur im Bedarfsfall entlang des Anschnitts vergrößert wird. Die Ausnehmung kann auch von zwei Öffnungen im Bereich der Nasenlöcher oder mittels mindestens einem ringförmig perforierten Einschnitt in die Maske gebildet werden. Um die Nasenöffnung zur Wirkung zu bringen, wird der von der Perforation eingeschlossen Maskenteil herausgelöst. Im Fall einer linienförmigen Perforation kann die Maske entlang der Linie geöffnet werden und so einen Durchtritt für die Nase ausbilden.

Die Ausnehmung für den Mund ist vorzugsweise derart gestaltet, dass sie einen insbesondere weit geöffneten Mund frei gibt, um umgehindert Zahnbehandlungen am Maskenträger durchführen zu können.

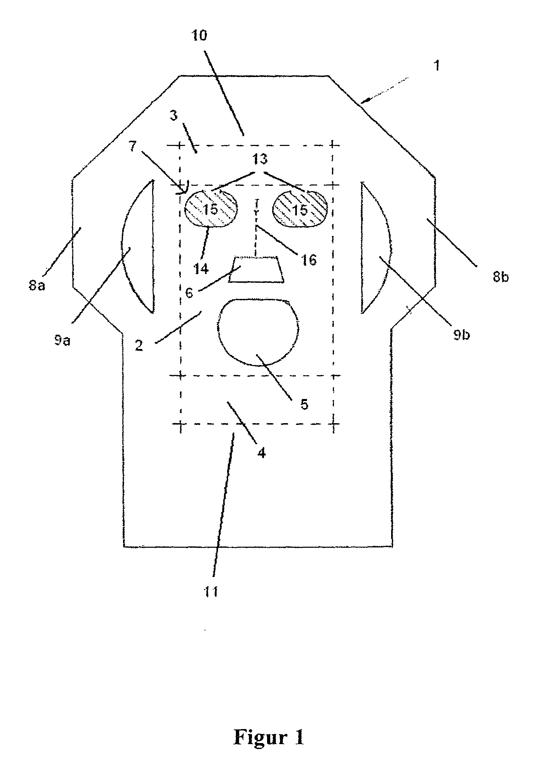

Nachfolgend wird die Erfindung anhand von Figur 1 näher erläutert.

Figur 1 zeigt eine erfindungsgemäße Gesichtsmaske (1), die vollständig aus ein- oder mehrlagigem Zellstoff-Gewebe besteht.

Die Gesichtsmaske (1) umfasst eine zentrale Augen-, Nasen-, Mund- und Wangenpartie, nachfolgend lediglich als Zentrale Partie (2) bezeichnet sowie eine davon abgesetzte Stirnpartie (3) und eine Kinnpartie (4). In der zentralen Partie (2) sind eine Ausnehmung für den Mund (5), eine trapezförmige Ausnehmung für die Nase (6) sowie zwei verschließbare Ausnehmungen (7) für die Augen des Maskenträgers angeordnet. Seitlich an der zentralen Partie der Gesichtsmaske (1) sind Fortsätze (8 a, 8 b) angeordnet, die Durchgänge (9 a, b) zur Aufnahme der Ohren aufweisen. Die Durchgänge (9 a, b) sind teilkreisförmig ausgestaltet, wobei die Kreisbögen zu den äußeren Rändern der seitlichen Fortsätze (8 a, b) weisen, damit die Durchgänge (9 a, b) komfortabel über die Ohren des Maskenträgers gezogen werden können.

Die Gesichtsmaske (1) weist darüber hinaus angrenzend an die Stirnpartie (3) der Gesichtsfläche einen flächigen Fortsatz (10) zur Abdeckung zumindest des Haaransatzes des Maskenträgers auf. Angrenzend an die Kinnpartie (4) schließt sich ein flächiger Fortsatz (11) zur Abdeckung der Halspartie an.

Die Ausnehmungen für die Augen (7) werden durch eine auf einer Teillänge (13) unterbrochenen Stanzung (14) in der Gesichtsmaske gebildet. Der von der Stanzung (14) umgebene Bereich ist über die nicht ausgestanzte Teillänge (13) an dem Rest der Gesichtsmaske (1) scharniert. Infolge dessen lassen sich die von der Stanzung (14) umgebenen Verschlussklappen (15) bedarfsweise in die in der Figur 1 dargestellte Schließ oder auch eine Öffnungsposition bewegen.

Eine weitere Besonderheit stellt der oberhalb der trapezförmigen Ausnehmung (6) für die Nase befindliche Anschnitt (16) in der Gesichtsmaske (1) dar, der sich bis auf Höhe der Augenpartie erstreckt. Der Anschnitt (16) erlaubt bei Bedarf eine Vergrößerung der Ausnehmung (6) für die Nase, in dem die trapezförmige Öffnung durch Einreißen des Materials der Gesichtsmaske längs des Anschnitts (16) vergrößert wird.

Die Ausnehmung (5) für den Mund ist derart gestaltet, dass sie einen geöffneten Mund für eine ungehinderte zahnärztliche Behandlung freigibt. Für andere Anwendungsfälle ist es selbstverständlich möglich, die Ausnehmung für den Mund kleiner zu gestalten.

Die in Figur 1 dargestellte Gesichtsmaske (1) ist in vorteilhafter Ausgestaltung der Erfindung einstückig vollständig aus einem Zellstoffvlies hergestellt und kann daher nach einmaligem Gebrauch als Einwegprodukt umweltfreundlich entsorgt werden.

**Bezugszeichenliste**

| Nr. | Bezeichnung |
|---|---|
| 1. | Gesichtsmaske |
| 2. | zentrale Partie |
| 3. | Stirnpartie |
| 4. | Kinnpartie |
| 5. | Ausnehmung für Mund |
| 6. | Ausnehmung für Nase |
| 7. | Ausnehmungen für Augen |
| 8. | seitliche Fortsätze |
| 9. | Durchgänge |
| 10. | Fortsatz Haaransatz |
| 11. | Fortsatz |
| 12. | Fortsatz Halspartie |
| 13. | Teillänge |
| 14. | Stanzung |
| 15. | Verschlussklappen |
| 16. | Anschnitt |

## Patentansprüche

1. Gesichtsmaske umfassend eine zentrale Augen-, Nasen-, Mund - und Wangenpartie sowie eine Stirn- und Kinnpartie zur Abdeckung der Gesichtsfläche eines Maskenträgers mit Ausnehmungen zumindest für Mund und Augen sowie einer Halterung zur Befestigung der Gesichtsmaske am Kopf des Maskenträgers, **dadurch gekennzeichnet,**
- **dass** die Gesichtsmaske (1) seitlich neben der Augen-, Nasen-, Mund - und Wangenpartie (2) Durchgänge (9a,9b) zur Aufnahme der Ohren des Maskenträgers aufweist,
- **dass** die Gesichtsmaske aus einem flexiblen Material besteht,
- **dass** an den Ausnehmungen (7) für die Augen jeweils ein Verschluss (15) angeordnet ist und
- **dass** die Ausnehmung (7) für das Auge und der Verschluss (15) durch eine das Auge umgebende, auf einer Teillänge unterbrochene Stanzung (14) im Bereich der Augenpartie der Gesichtsmaske (1) gebildet sind.

2. Gesichtsmaske nach Anspruch 1, **dadurch gekennzeichnet, dass** sich die Durchgänge (9a,9b) zur Aufnahme der Ohren längs der Symmetrieachse der abgedeckten Gesichtsfläche (2,3,4) von der Augenpartie bis zur Mundpartie erstrecken.

3. Gesichtsmaske nach Anspruch 1 oder 2, **dadurch gekennzeichnet, dass** die Durchgänge (9a,9b) zur Aufnahme der Ohren teilkreisförmig ausgestaltet sind.

4. Gesichtsmaske nach einem der Ansprüche 1 bis 3, **dadurch gekennzeichnet, dass** die Durchgänge (9a,9b) zur Aufnahme der Ohren in seitlich an die Gesichtsfläche (2,3,4) der Gesichtsmaske (1) ansetzenden flächigen Fortsätzen (8a,8b) angeordnet sind.

5. Gesichtsmaske nach einem der Ansprüche 1 bis 4, **dadurch gekennzeichnet, dass** die Gesichtsmaske (1) angrenzend an die Stirnpartie (3) einen flächigen Fortsatz(10) zur Abdeckung zumindest des Haaransatzes aufweist.

6. Gesichtsmaske nach einem der Ansprüche 1 bis 5, **dadurch gekennzeichnet, dass** die Gesichtsmaske (1) angrenzend an die Kinnpartie (4) einen flächigen Fortsatz (11) zur Abdeckung der Halspartie aufweist.

7. Gesichtsmaske nach einem der Ansprüche 1 bis 6, **dadurch gekennzeichnet, dass** die Gesichtsschutzmaske über mindestens eine Ausnehmung (6) für die Nase verfügt.

8. Gesichtsmaske nach Anspruch 7, **dadurch gekennzeichnet, dass** die Ausnehmung für die Nase eine trapezförmige Öffnung ist.

9. Gesichtsmaske nach Anspruch 8, **dadurch gekennzeichnet, dass** sich von der trapezförmigen Öffnung in Richtung der Symmetrieachse ein Ein-/oder Anschnitt (16) in die Gesichtsmaske (1) bis auf Höhe der Augenpartie erstreckt.

10. Gesichtsmaske nach einem der Ansprüche 1 bis 9, **dadurch gekennzeichnet, dass** die Ausnehmung (5) für den Mund derart gestaltet ist, dass sie einen geöffneten Mund freigibt.

11. Gesichtsmaske nach einem der Ansprüche 4 bis 10, **dadurch gekennzeichnet, dass** die Fortsätze (8a,8b,10,11) der Gesichtsmaske (1) einstückig mit dem Rest der Gesichtsmaske (2,3,4) ausgebildet sind.

12. Gesichtsmaske nach einem der Ansprüche 1 bis 11, **dadurch gekennzeichnet, dass** sie aus Zellstoffmaterial besteht.

13. Gesichtsmaske nach Anspruch 12, **dadurch gekennzeichnet, dass** sie aus einem mindestens einlagigen Zellstofftuch besteht.

14. Verwendung der Gesichtsmaske nach einem der Ansprüche 1 bis 13 als Spritzschutz bei kosmetischen und zahnärztlichen Behandlungen.
